# EUROPEAN PATENT APPLICATION

(11) **EP 2 656 782 A1**
(43) Date of publication of application: **30.10.2013**
(21) Application number: 11851174.0
(22) Date of filing: 19.12.2011
(51) Int. Cl.: A61B 5/00

(54) **DEVICE WHICH COMPRISES A PHYSICAL ACTIVITY AND POSITION SENSOR, A PERIPHERAL TEMPERATURE SENSOR AND A LIGHT SENSOR FOR PROVIDING INFORMATION RELATING TO THE STATE OF THE CIRCADIAN SYSTEM**

(30) Priority: 21.12.2010 ES 201031894
(71) Applicant: Universidad de Murcia, 30100 Espinardo, Murcia (ES)
(72) Inventor: SARABIA CARRAZO, Juan Antonio, E-30100 Murcia (ES); MARTINEZ NICOLAS, Antonio, E-30100 Murcia (ES); MADRID PEREZ, Juan Antonio, E-30100 Murcia (ES); ROL DE LAMA, María Angeles, E-30100 Murcia (ES); ORTIZ TUDELA, Elisabet, E-30100 Murcia (ES)
(74) Representative: Carvajal y Urquijo, Isabel
(86) International application number: PCT/ES2011/070877
(87) International publication number: WO 2012/085315

(57) **Abstract**

The invention is a device consisting of at least one body position and activity sensor, at least one peripheral temperature sensor and at least one light sensor, configured to provide information on the circadian system status and the sleep-wake status of an individual based on the data obtained from said sensors. It can be placed on the wrist of the subject, or on the subject's arm. The device may also include a blood pressure sensor. This device may be used in general studies of the human circadian system and, more specifically, for the study of sleep-wake and blood pressure rhythms.

## Description

### FIELD OF THE INVENTION

The invention concerns a device that constitutes a body position and activity sensor, a peripheral temperature sensor and a light sensor. This device may be used in general studies of the human circadian system and, more specifically, for the study of sleep-wake and blood pressure rhythms.

### BACKGROUND OF THE INVENTION

Biological rhythms are biological variables that oscillate on a regular basis and have a specific period. Circadian rhythms have a period of approximately 24 hours.

The circadian system consists of all structures responsible for generating the different biological rhythms and synchronizing them to environmental cycles. Its main component is a pacemaker made up by a group of neurons located in the hypothalamus (the suprachiasmatic nucleus). This pacemaker uses nervous and hormonal signals to control the circadian rhythms of the rest of the body.

In order to evaluate the functioning of the circadian system correctly, one or more biological rhythms controlled by the circadian pacemaker must be monitored for the longest period of time possible, the ideal being one or more weeks. Traditional methods of measurement involve invasive procedures, such as the determination of blood melatonin and cortisol levels; uncomfortable methods, such as recording the body's core temperature with a rectal probe; and procedures in which the determination of one variable alters one of the most important rhythms, the sleep-wake rhythm, as it requires the active cooperation of the patient or experimental subject, as in the case of determining melatonin and cortisol levels in saliva or measuring tympanic temperature.

Its ease of use for recording and non-invasive nature have gradually led to the increased use of actimetry for evaluating the human circadian system. Actimetry allows for determining whether the subject is asleep or engaged in various intensities of activity based on the movements within a certain area of the body, usually the wrist. However, the use of actimetry to evaluate the circadian system presents certain disadvantages, among them the fact that it is difficult to differentiate between the initial rest period associated with sleep and when the subject has removed the sensor, for example, to take a bath or shower, or an increase in movement associated with car trips or even the movement of a partner in bed.

Recently, the peripheral temperature rhythm has been suggested as a marker rhythm, given that it has obvious advantages in terms of measurement and is also a circadian system indicator (Sarabia, J.A. et al., Circadian rhythm of wrist temperature in normal-living subjects. A candidate of new index of the circadian system. PhysiolBehav. 2008; 95(4):570-80).

The body's peripheral temperature follows a circadian profile in such a way that the temperature increases as a prelude to sleep and remains high for the rest of the night. Upon awakening, it drops sharply, and its levels remain low throughout the day. In fact, it is this rhythm that instigates the changes in the core body temperature, which has been used for decades as a reliable marker rhythm in chronobiological studies. This is due to the fact that vasodilation of the extremities and the subsequent increase in peripheral temperature is what triggers the drop in core temperature. Recent studies have identified a direct relationship with melatonin production, making it an indirect method of determining melatonin levels in the body.

The problem this technique poses is obtaining information on the status of the human circadian system in a non-invasive manner and with more than one variable. The solution proposed by this invention is a device that jointly integrates and processes the information generated by a body position and activity sensor, a peripheral temperature sensor worn on the wrist and a light sensor.

### DESCRIPTION OF THE INVENTION

The invention is a device consisting of at least one body position and activity sensor, at least one peripheral temperature sensor and at least one light sensor, configured to provide information on the circadian system status and the sleep-wake status of an individual based on the data obtained from said sensors.

In terms of the present invention, "circadian system" is understood to mean the set of biological variables in an individual, which undergo regular oscillations over a 24-hour period. The invented device stores and processes information about peripheral temperature of the wrist, body position and activity and environmental light parameters. All sensors can be programmed to record information over a wide range of time intervals. The device's sampling interval can be programmed, which makes it more useful in clinical settings.

The body position and activity sensor is a 3-axis accelerometer with a measurement range of ± 3g that provides information on the position of the X, Y and Z axes. These data are used to define 2 variables: body position and motor activity. The values for body position are defined with regard to the X axis (perpendicular to the earth's surface) and with a range between 0 and 90°, with 0° representing a horizontal position and 90° representing the maximum vertical position. Motor activity is defined as the number of degrees of change in the position of the activity sensor as compared to the recording taken immediately before, and the average acceleration to which the sensor has been subjected during each recording interval.

The light sensor records exposure to different light intensities the subject receives (measured in luxes), evaluated at very short time intervals. Light intensity is the most important external synchronization signal, and is responsible for setting the time of the circadian clock and preventing it from falling behind.

A preferred implementation is a device based on this invention, which is placed on the subject's wrist.

Another implementation of this invention is a device which also consists of at least one blood pressure sensor and that is programmed to provide information about the status of a subject's blood pressure.

This device improves the diagnostic accuracy of changes in circadian blood pressure rhythms, enabling the subject's rest period to be objectively determined based on position changes. Existing devices for blood pressure measurement arbitrarily establish rest periods, and therefore do not provide a reliable measurement of the changes in blood pressure values when the subject lies down or stands up. These changes are very important for the diagnosis of hypertension.

A preferred implementation is the use of the invention in a way in which the aforementioned peripheral temperature and light sensors are positioned on the wrist of the subject, while the body position and activity and blood pressure sensors are placed on the subject's arm.

Yet another implementation of the invention is a procedure to determine the circadian system status and the sleep-wake status of the subject that consists of:
a) Obtaining peripheral temperature, motor activity per minute and body position values for the subject.
b) Drawing conclusions as to the circadian system status and the sleep-wake status of a subject based on the changes in the values from step a).

A preferred implementation involves a procedure using the invention to obtain information on blood pressure and hypertension, characterized by the fact that in step a) blood pressure measurements are also taken for the subject, and in step b) the blood pressure values are compared, along with the rest of the values, to determine any changes in blood pressure upon lying down or standing up.

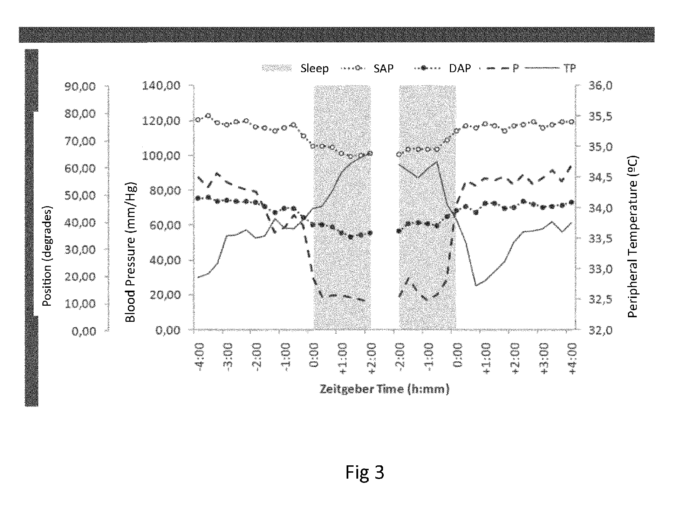

Figure 3 shows the inverse proportional relationship that exists between the peripheral temperature pattern and blood pressure, and the direct relationship between the position measured on the arm and blood pressure.

### BRIEF DESCRIPTION OF THE FIGURES

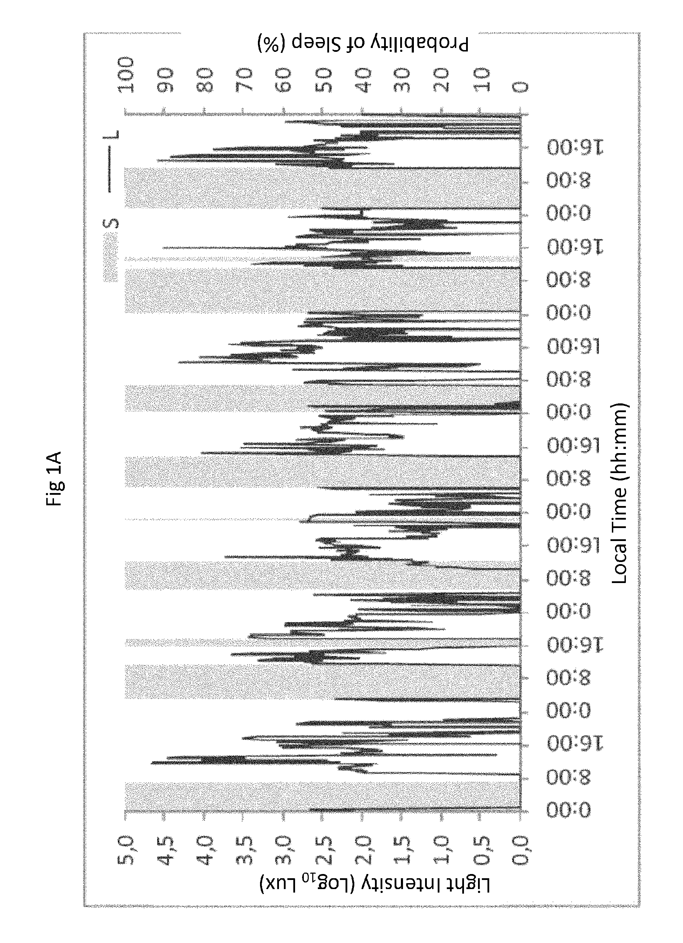

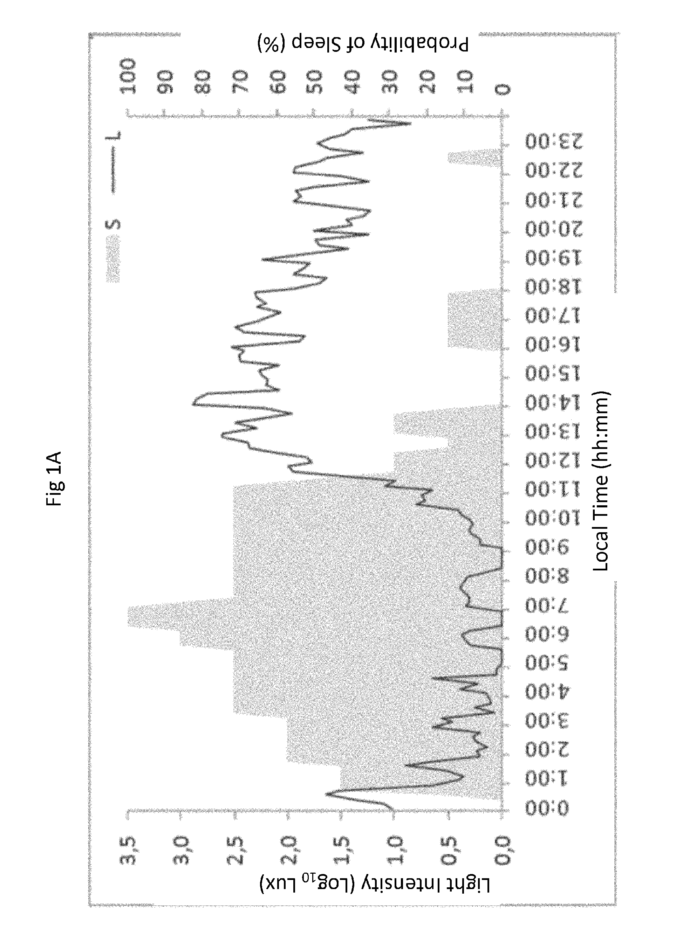

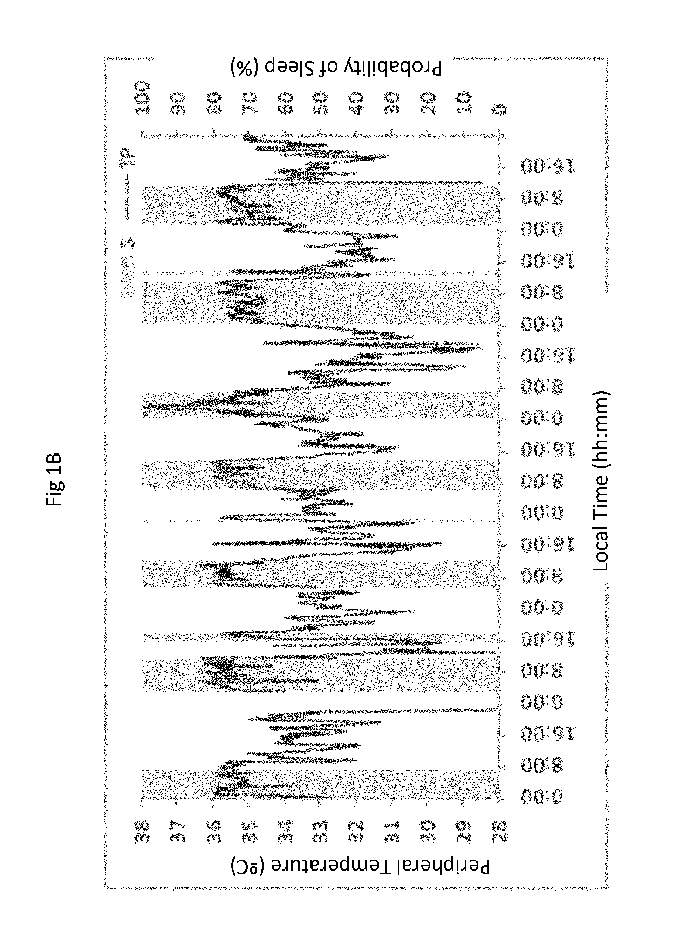

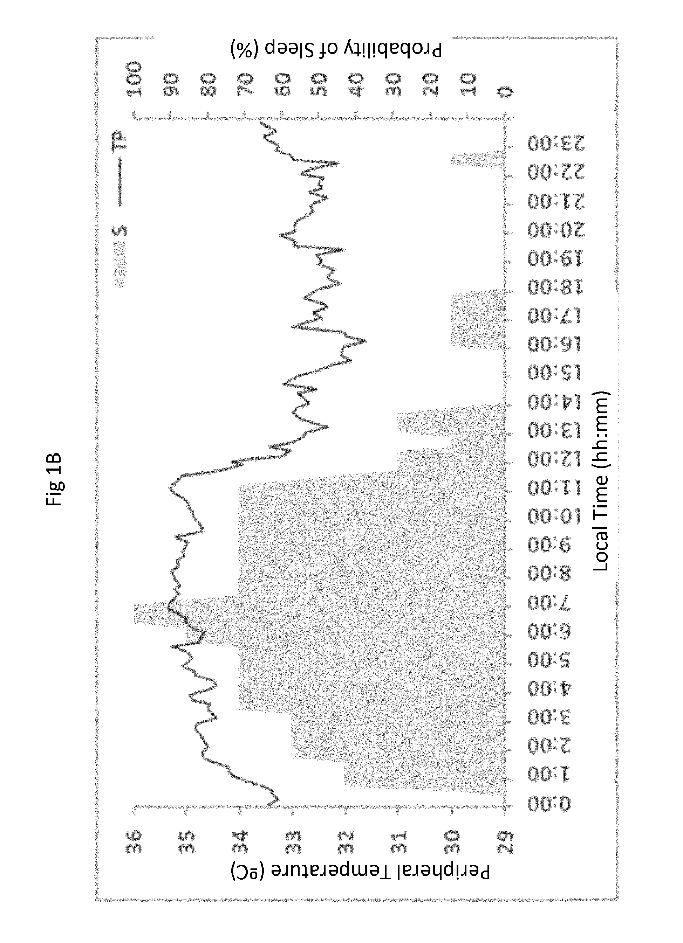

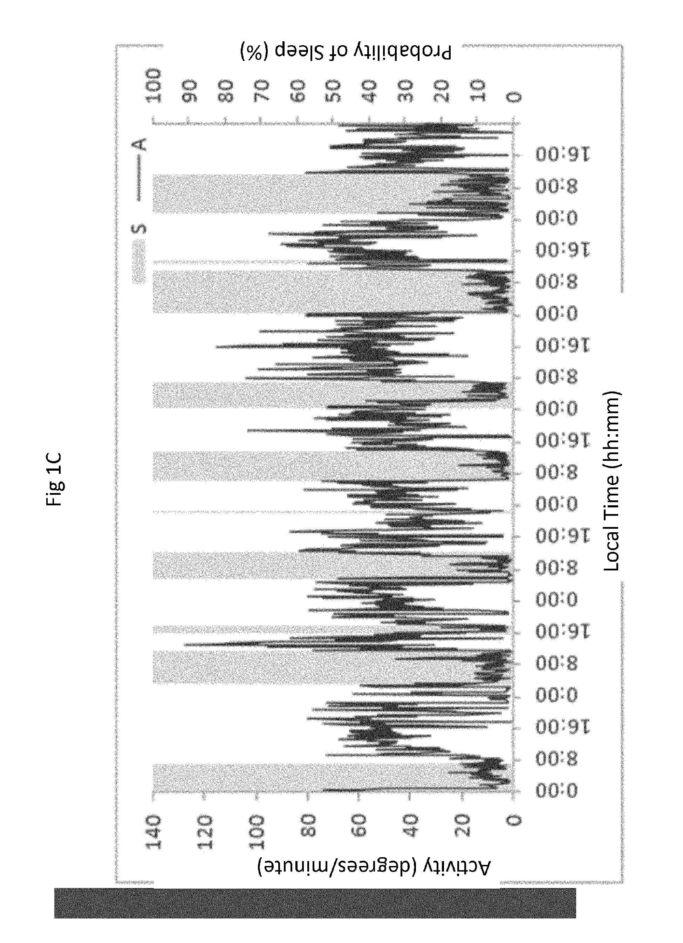

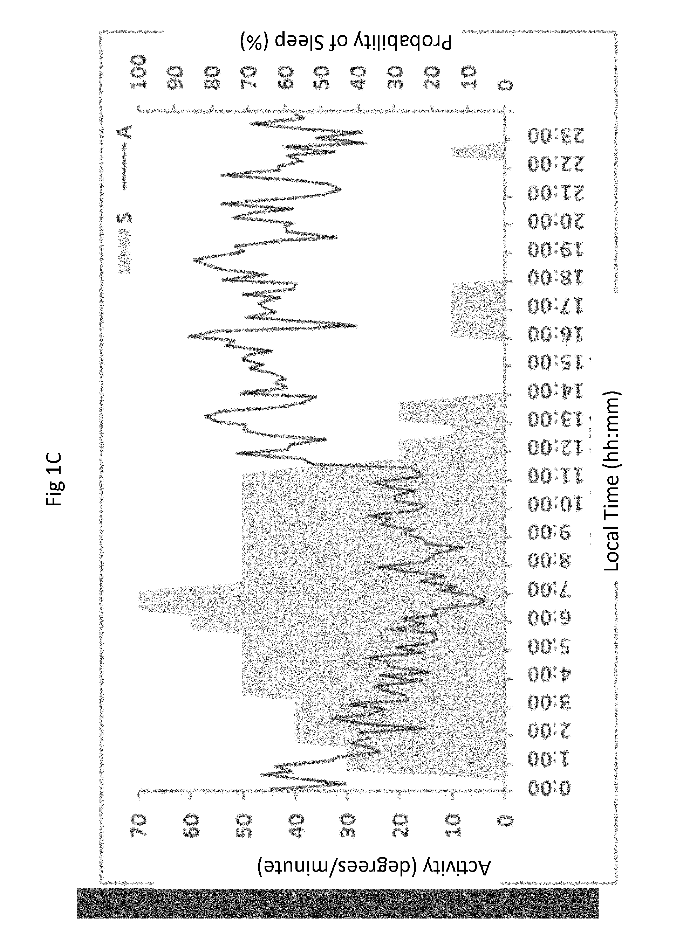

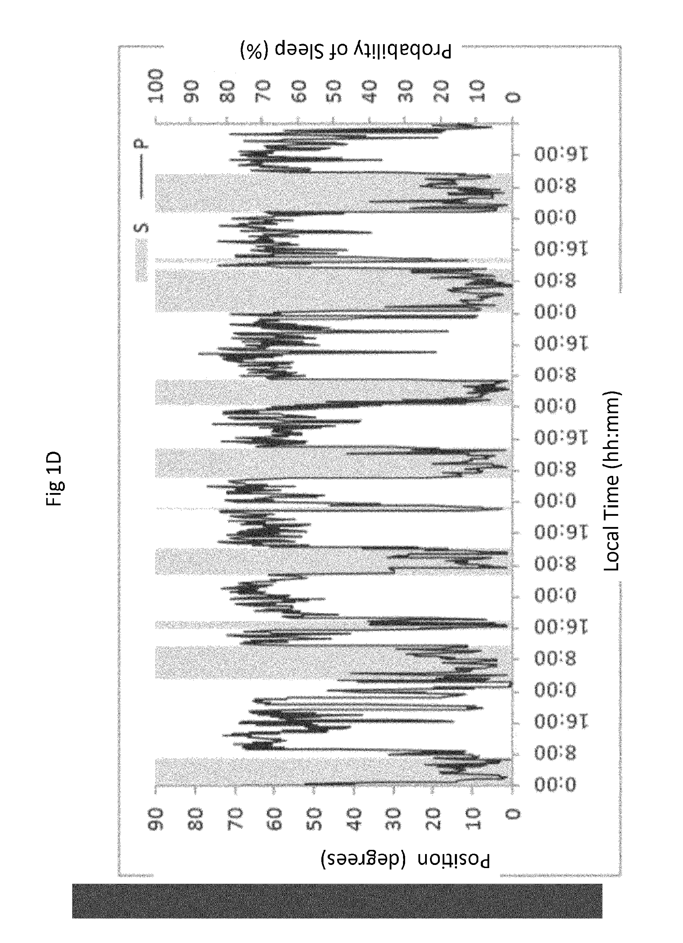

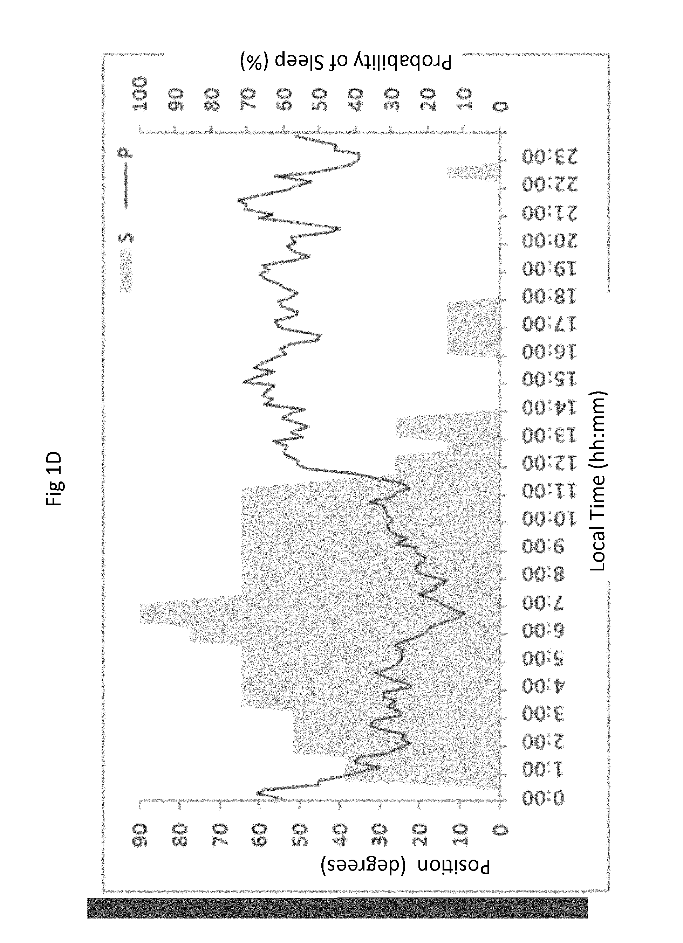

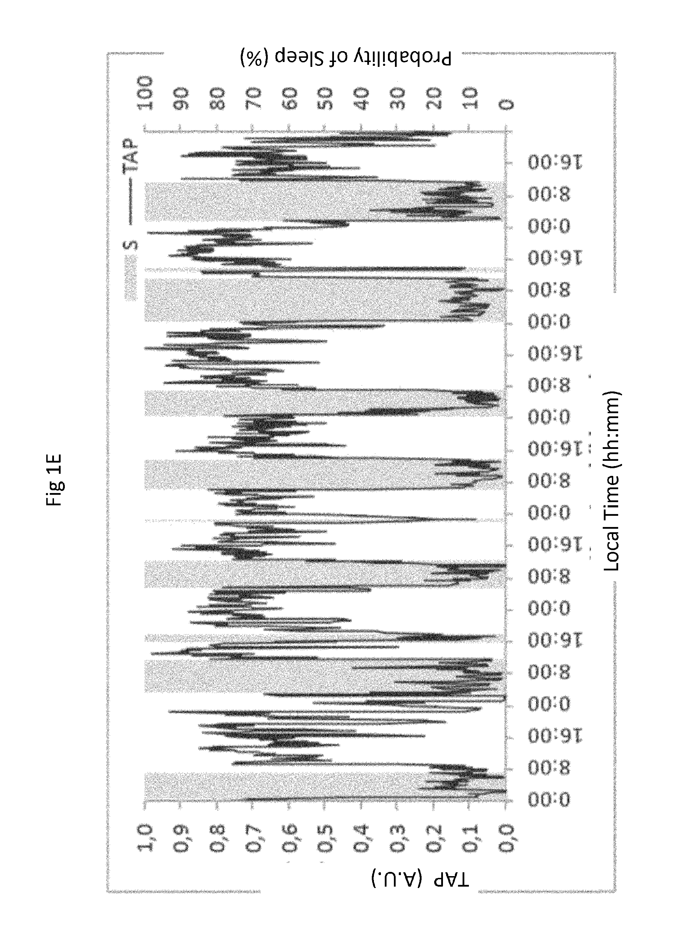

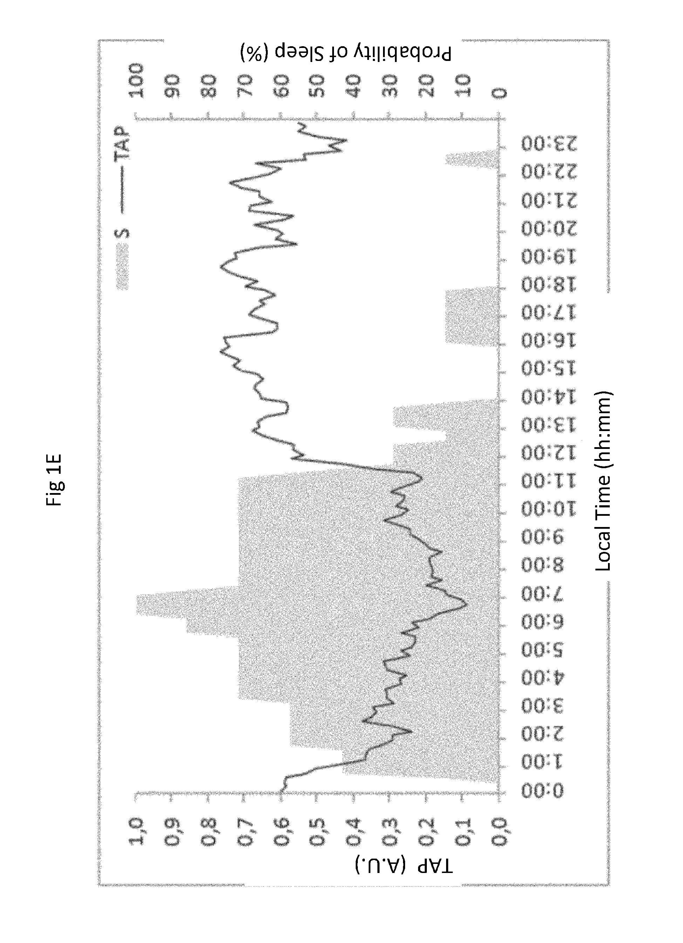

**Figure 1****.** Seven-day recording and average daily wave of the main recorded variables; light intensity (Figure 1A), peripheral temperature (Figure 1B), motor activity (Figure 1C), body position (C) (Figure 1 D) and the integrated TAP variable (D) (Figure 1 E) for a representative subject. The grey shaded area shows the subject's sleep periods, and the values of the recorded variables are shown by the solid line.

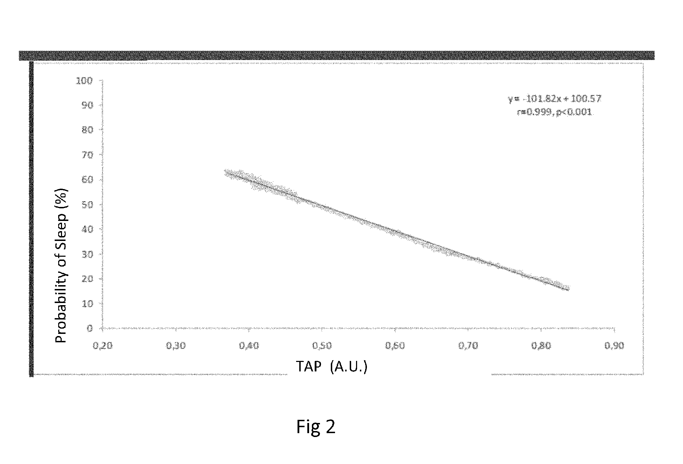

**Figure 2****.** Comparison of the TAP variable and sleep probability. The vertical axis shows the probability of sleep, expressed in decimal form. Accordingly, a value of 1 indicates that 100% of the subjects were asleep, and a value of 0 indicates that they were all awake.
**Figure 3****.** Representation of wrist peripheral temperature (solid line), body position (dotted line), systolic blood pressure (empty circles) and diastolic blood pressure (solid circles). The four hours prior to going to bed and the two hours afterwards are shown, as are the two hours prior to getting up and the four hours afterwards. The shaded area represents the rest phase. The data are adapted to a synchronizer (*zeitgeber*), which in this case is sleep onset (left) and sleep end (right). Therefore, the horizontal axis represent time in relation to the start and end of sleep (*zeitgeber time*).

### EXAMPLES OF THE INVENTION

### Example 1. Recording wrist peripheral temperature, motor activity and body position variables, as well as blood pressure, in various subjects.

Data was recorded for 37 subjects, who wore sensors consecutively for one week. All subjects were young people between 20 and 30 years of age. The subjects lived a normal life, as the technique is precisely intended to study the status of the circadian rhythm in persons without the need to modify their daily habits. The sensors were only removed to allow the subjects to shower.

### Example 2. Processing of physiological data

TAP is a modular variable and consists of 3 separate variables: peripheral temperature (T), motor activity per minute (A) and body position (P). The TAP variable was obtained in the following manner: the first step consisted of standardizing these variables as values between 0 and 1. To do this, the 95^{th} and 5^{th} percentiles were calculated for each variable and participating subject. All values above the 95^{th} percentile were considered to be 1, and all values below the 5^{th} percentile were considered to be 0. For intermediate values between the 5^{th} and 95^{th} percentile, the proportional value between 0 and 1 was calculated.

So that the highest values were indicative of greater levels of activity in all three variables, the standardized values were inverted for the peripheral temperature (T' norm = 1 - T norm). Once the data had been standardized, the sum of the 3 variables was then calculated. Thus, the TAP variable had a maximum value of 3 (maximum level of activity) and a minimum value of 0 (complete state of rest).

After standardizing the variables and calculating the TAP variable, the match rates were calculated for each of the variables studied (including TAP) and the daily sleep diary (with values of 0 whenever the individual claimed to be awake, and values of 1 when he/she reported being asleep), in order to ascertain whether our TAP variable improved the ability to predict each of the variables separately. These results are shown in Figure 1.

## Claims

1. Device that consists of at least one body position and activity sensor, at least one peripheral temperature sensor and at least one light sensor, configured to provide information about the circadian system status and the sleep-wake status of an individual based on the data obtained from said sensors.

2. Device as described in claim 1, **characterized by** the fact that this device is placed on the person's wrist.

3. Device as described in claim 1, **characterized by** the fact that it also includes at least one blood pressure sensor and is configured to provide information on the status of a person's blood pressure.

4. Device as described in claim 3, **characterized by** the fact that these peripheral temperature and light sensors are placed on the person's wrist and said body position and activity sensors and blood pressure sensor are placed on the person's arm.

5. Procedure to determine the circadian system status and the sleep-wake status of a person, consisting of:
a) Obtaining the peripheral temperature, motor activity per minute and body position values for an individual.
b) Drawing conclusions about the circadian system status and the sleep-wake status of an individual based on the changes in the values resulting from step a).

6. Procedure as described in claim 5 to obtain information about blood pressure and hypertension, **characterized by** the fact that in step a) blood pressure values are also recorded for said individual, and that in step b) the blood pressure values are compared to the rest of the values to determine any changes in blood pressure upon lying down or standing up.
